# EUROPEAN PATENT APPLICATION

(11) **EP 1 568 360 A1**
(43) Date of publication of application: **31.08.2005**
(21) Application number: 03772728.6
(22) Date of filing: 12.11.2003
(51) Int. Cl.: A61K 9/127, A61K 47/42, A61K 45/00, A61K 47/24, A61K 47/34, A61P 35/00

(54) **LIPOSOME**

(30) Priority: 15.11.2002 JP 2002332825
(71) Applicant: NIPRO CORPORATION, 531-8510 (JP)
(72) Inventor: KAI, Toshiya, c/o NIPRO CORPORATION, Osaka-shi, Osaka 531-8510 (JP); YOKOE, Junichi, c/o NIPRO CORPORATION, Osaka-shi, Osaka 531-8510 (JP); AZUMA, Yuko, c/o NIPRO CORPORATION, Osaka-shi, Osaka 531-8510 (JP); SATO, Makoto, c/o NIPRO CORPORATION, Osaka-shi, Osaka 531-8510 (JP); KIMURA, Toshikiro, Okayama-shi, Okayama 700-0085 (JP); HIGAKI, Kazutaka, Okayama-shi, Okayama 703-8281 (JP)
(74) Representative: Albrecht, Thomas
(86) International application number: PCT/JP2003/014405
(87) International publication number: WO 2004/045583

(57) **Abstract**

The present invention provides a liposome to which a polyalkylene glycol and albumin are bonded having an excellent retention in blood.

## Description

### Technical Field

The present invention relates to a liposome having an excellent retention in the blood.

### Background Art

Liposome is a lipid vesicle comprising lipid bilayer membrane and has been studied mostly as a carrier for various drugs for injections. Recently, its application as a carrier for gene in gene therapy has been actively studied as well. Amphotericin B which is an antimycotic agent, doxorubicin and daunorubicin which are anticancer agent, and indium which is a contrast agent have been put on the market as liposome preparations already (Troy O. Harasym, Marcel B. Bally, Paul Tardi, "Clearance properties of liposomes involving conjugated proteins for targeting", Advanced Drug Delivery Reviews, 1998, vol. 32, p. 99-118).

Although liposome is constituted from biocompatible lipid, it has been known to quickly disappear from blood because it is recognized as a foreign body by immune system after its intravenous administration. Accordingly, it is the biggest disadvantage that effect of the drug after administration is not long-acting. In order to solve such disadvantages, a method where glycoprotein or glycolipid are chemically modified on the surface of liposome, a method where a glucuronic acid derivative is bonded thereto, etc. have been reported, but none of them has been actually used for pharmaceutical preparations.

In the meanwhile, since 1990's, there has been widely known a technological method where recognition by immune system is avoided by chemical modification of liposome surface with hydrophilic polyethylene glycol and action of the drug is sustained by improving the retention of liposome in the blood. Such technology method is also applied to the above-mentioned commercially available liposome preparations. Moreover, such method has been also widely investigated for applying to anticancer drugs such as cisplatin, vincristine and camptothecin (Naoto Oku, Yoshihiro Tokudome, Tomohiro Asai and Hideo Tsukada, "Evaluation of Drug Targeting Strategies and Liposomal Trafficking", *Current Pharmaceutical Design,* 2000, vol. 6, p. 1669-1691).

In addition, modification of the surface of liposome has also been briskly conducted in studies for targeting of drugs to cancer cells or hepatocytes, and there have been reported a method where cancer cells are recognized by bonding to antibody (Troy O. Harasym, Marcel B. Bally, Paul Tardi, "Clearance properties of liposomes involving conjugated proteins for targeting", Advanced Drug Delivery Reviews, 1998, vol. 32, p. 99-118) or transferrin, a method for incorporation into hepatocytes by bonding to various sugar chains, etc. In chemical modifications as such, utilization of albumin as a spacer has been reported (Shuji Kojima, Yusuke Sogawa, Yoshika Tajiri and Noboru Yamaki, "Investigations in Suppression and Promotion of Incorporation of Glycoprotein-Bonded Liposome by Modification with Sialic Acid into Reticuloendothelial System", *Drug Delivery System,* 2002, vol. 17-1, p. 63-68).

### Disclosure of the Invention

An object of the present invention is to provide a liposome of which retention in blood is much more improved.

In order to achieve the above-mentioned object, the present inventors have carried out intensive studies and found that, when polyethylene glycol (hereinafter, may be abbreviated as PEG) and albumin are simultaneously bonded to a liposome, retention of the liposome in blood is synergistically improved. It has been also found that, even in a modifying amount of PEG by which almost no effect is noted in the retention in blood through modification of PEG only, an apparent effect is noted when albumin is used together for the modification. Incidentally, there has been no report yet for bonding of PEG and albumin to the surface of liposome.

Thus, the present invention relates to the followings.
(1) A liposome to which a polyalkylene glycol and albumin are bonded.
(2) The liposome according to the above (1), wherein a physiologically active ingredient is further contained.
(3) The liposome according to the above (2), wherein the physiologically active ingredient is a pharmaceutically active ingredient.
(4) The liposome according to the above (3), wherein the pharmaceutically active ingredient is an antitumor agent.
(5) A pharmaceutical composition containing the liposome mentioned in any one of the above (2) to (4).
(6) The pharmaceutical composition according to the above (5), which is an injection.
(7) A method for treatment of cancer, which comprises administering a pharmaceutical composition comprising a liposome to which a polyalkylene glycol and albumin are bonded and in which an antitumor agent is contained.
(8) Use of a liposome to which a polyalkylene glycol and albumin are bonded and in which a physiologically active ingredient is contained, for the extension of the in vivo retention time of the physiologically active ingredient.
(9) A process for the production of the liposome mentioned in claim 1, characterized in that,
   a liposome having a compound represented by the following formula (1) 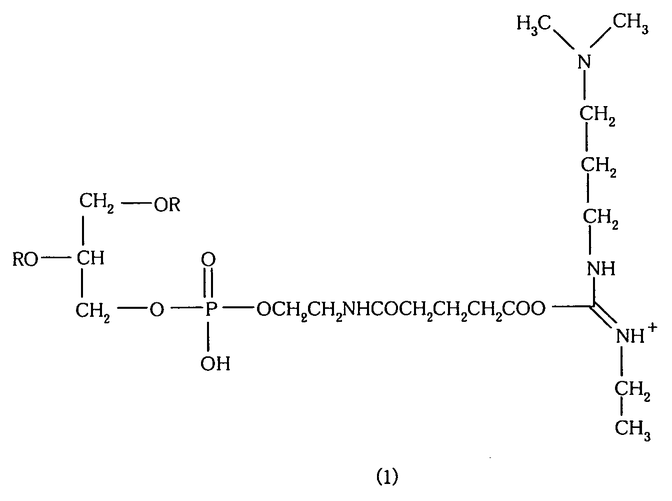 (wherein R is an acyl group derived from a fatty acid having 2 to 35 carbon atoms) as a constituent lipid is bonded to albumin;
   a liposome having a compound represented by the following formula (2) 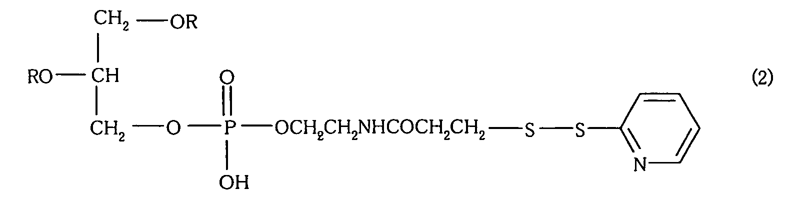 (wherein R has the same meaning as defined above) as a constituent lipid is bonded to a compound represented by the formula (3)

      (Alb-NH)-CO-CH₂-CH₂-SH (3)

      (wherein Alb-NH is a group formed by removing one hydrogen atom of the amino group from an albumin molecule represented by Alb-NH₂);
   a liposome having a compound represented by the following formula (4) 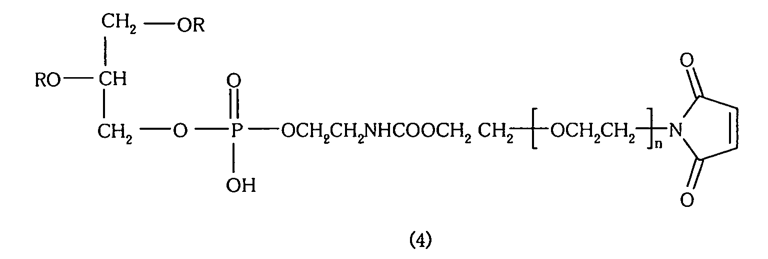 (wherein n is an integer of 5 to 100,000 and R has the same meaning as defined above) as a constituent lipid is bonded to a compound represented by the formula (5)

      (Alb-NH)-CO-CH₂-SH (5)

      (wherein Alb-NH has the same meaning as defined above);
   a compound represented by the following formula (6) 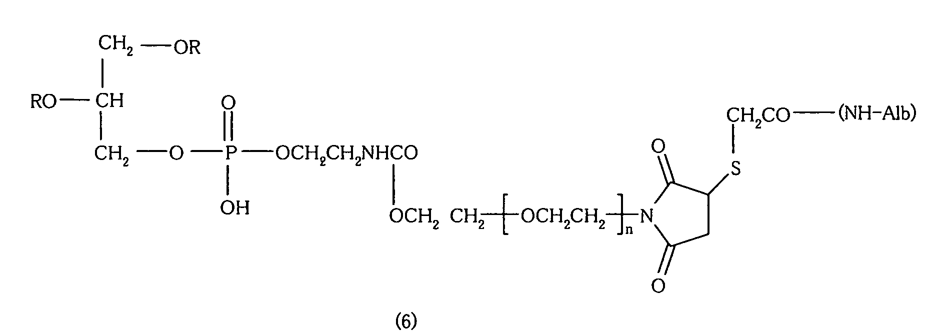 (wherein n, R and Alb-NH have each the same meaning as defined above) is inserted into a liposome;
   a liposome having the compound represented by the above formula (1) as a constituent lipid is bonded to a compound represented by the following formula (7) 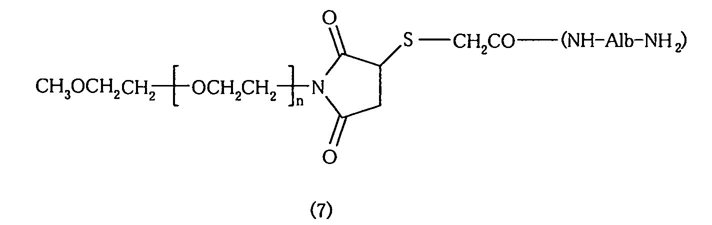 (wherein -NH-Alb-NH₂ is a group formed by removing one hydrogen atom from the one amino group of an albumin molecule represented by H₂N-Alb-NH₂, and n has the same meaning as defined above); or
   a liposome having the compound represented by the above formula (2) as a constituent lipid is bonded to a compound represented by the following formula (8) 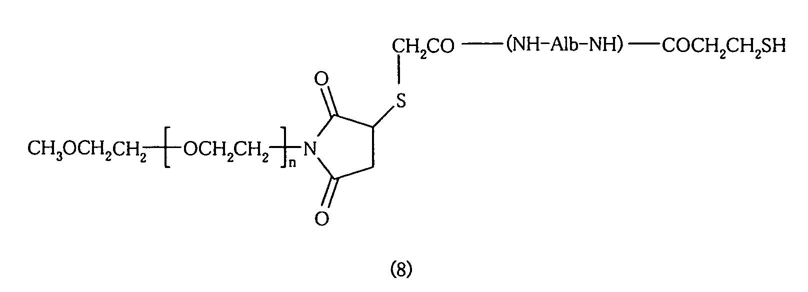 (wherein -NH-Alb-NH- is a group formed by removing each one hydrogen atom from the two amino groups of an albumin molecule represented by the formula H₂N-Alb-NH₂, and n has the same meaning as defined above).

R in the above compounds (1), (2), (4) and (6) is an acyl group derived from a saturated or unsaturated fatty acid having 2 to 35 carbon atoms. The above fatty acid has more preferably 6 to 18 carbon atoms and, most preferably, 8 to 16 carbon atoms. Specific examples of such fatty acids are octanoic acid (preferably, caprylic acid), decanoic acid (preferably, capric acid), dodecanoic acid (preferably, lauric acid), hexadecanoic acid (preferably, palmitic acid), octadecanoic acid (preferably, stearic acid) and monoenoic or polyenoic fatty acid thereof (preferably, oleic acid). Specific examples of such acyl group derived from the fatty acid are octanoyl group (preferably, capryloyl group), decanoyl group (preferably, caprinoyl group), dodecanoyl group (preferably, lauroyl group), hexadecanoyl group (preferably, palmitoyl group) and octadecanoyl group (preferably, stearoyl group) and there may be one or more double bond(s) therein (such as oleoyl group).

### Brief Description of Drawings

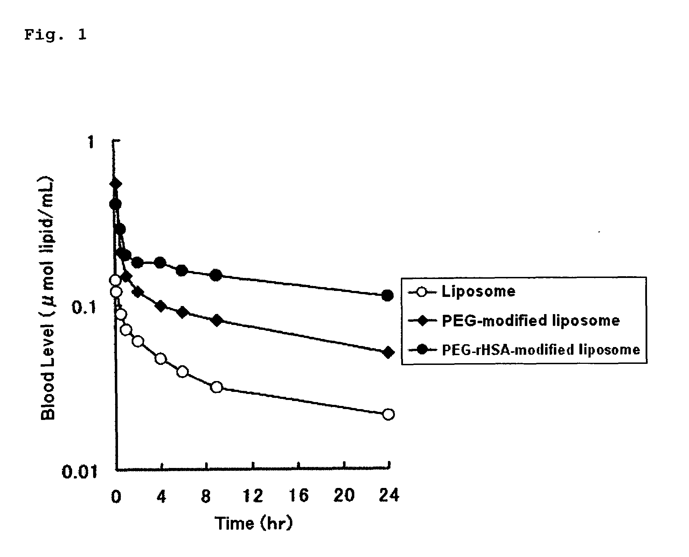

Fig. 1 is a drawing which shows the changes of concentration of liposome in blood with lapse of time measured in the Test Example.

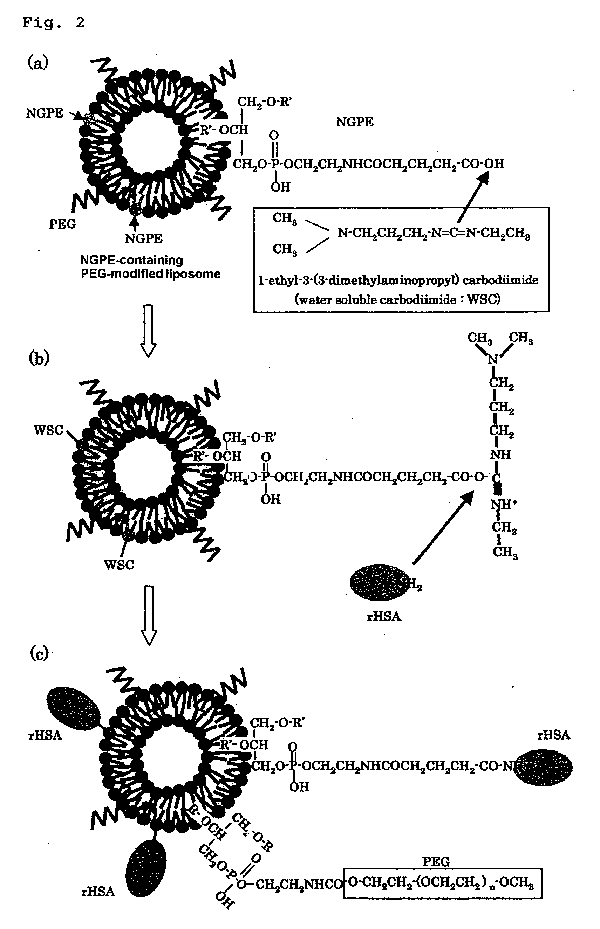

Fig. 2 is a schematic drawing which shows the process for production of liposome according to Method 1 of Example 1. (a) is a schematic drawing of PEG-modified liposome containing NGPE. R' in the drawing represents an oleoyl group. Only one of NGPE in the liposome as such is shown by a chemical formula. In the step (2) of Method 1 of Example 1, WSC is bonded to the carboxyl group shown by an arrow of NGPE, thereby to obtain a liposome as shown by a schematic drawing (b). In (b), although only one bond among the bonds of NGPE to WSC is shown in detail by way of chemical formula, other bonds are the same as well. After that, an amino group of human serum albumin (rHSA) is bonded to the carbonyloxy group shown by an arrow whereupon liposome as shown by a schematic drawing of (c) is prepared. In (c), although only one bond among the bonds of NGPE to rHSA is shown in detail by way of chemical formula, other bonds are the same as well. In addition, although only one bond among the bonds of DSPE to PEG is shown in detail by way of chemical formula, other bonds are the same as well. Incidentally, R represents a stearoyl group.

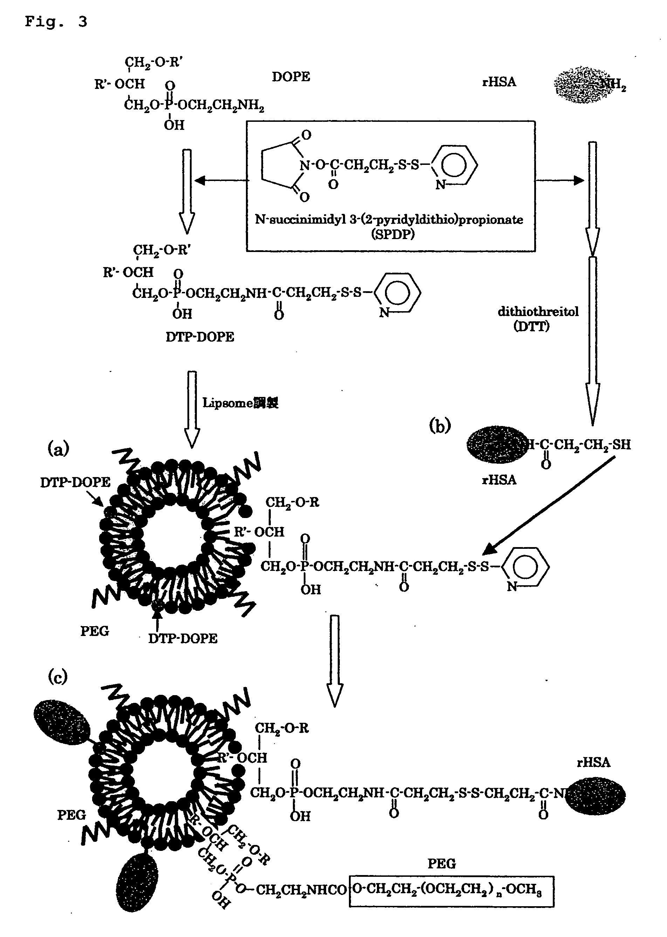

Fig. 3 is a schematic drawing which shows the process for production of a liposome according to Method 2 of Example 1. In the step (1) of Method 2 of Example 1, DOPE and SPDP are bonded to prepare DTP-DOPE and, in the step (2), a liposome shown by the schematic drawing (a) is produced by using such DTP-DOPE and PEG-bonded DSPE as constituent lipids. R' in the drawing represents an oleoyl group. Only one of DTP-DOPE in the liposome as such is shown by the chemical formula. When a mercapto group of the thiolated human serum albumin (rHSA) ((b) in the drawing) prepared in the step (3) is allowed to react with the dithio group of DTP-DOPE in said liposome, there is prepared the aimed liposome shown by the schematic drawing (c). In (c), although only one bond among the bonds of DTP-DOPE to rHSA is shown in detail by way of chemical formula, other bonds are the same as well. In addition, although only one bond among the bonds of DSPE to PEG is shown in detail by way of chemical formula, other bonds are the same as well. Incidentally, R in the drawing represents a stearoyl group.

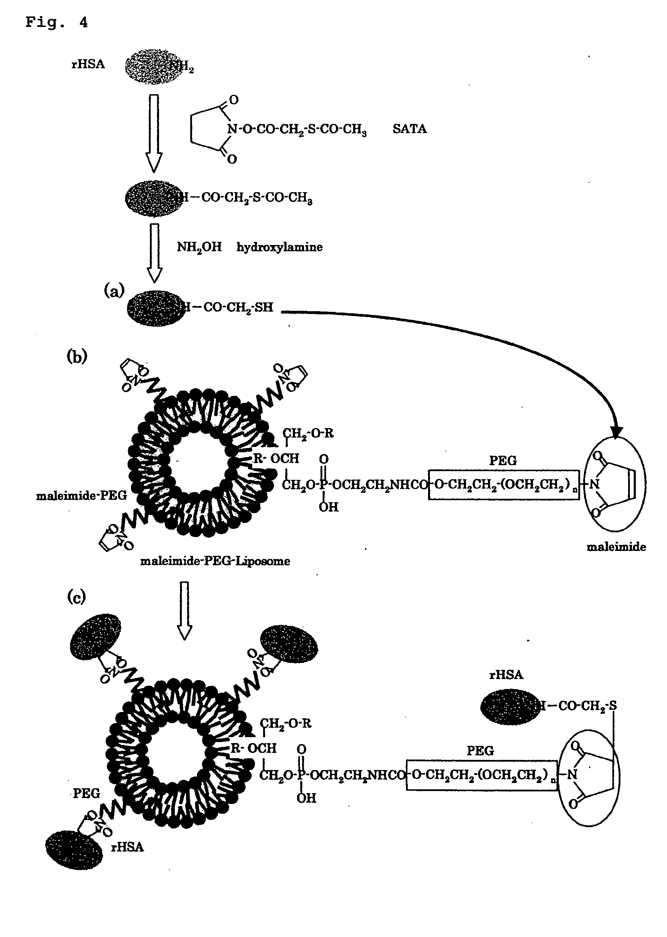

Fig. 4 is a schematic drawing which shows the process for production of a liposome according to Method 1 of Example 2. (a) is a schematic drawing of thiolated rHSA prepared according to Method 1 (2) of Example 2. (b) is a schematic drawing of a maleimide-PEG-modified liposome prepared according to Method 1(1) of Example 2. In (b), only one maleimide-PEG-DSPE in the liposome is shown by the chemical formula. When the thiolated rHSA shown by the schematic drawing (a) is reacted with the liposome shown by the schematic drawing (b), the aimed liposome shown by the schematic drawing (c) is obtained. Here, although only one bond among the bonds of PEG to rHSA via a maleimide group is shown in detail by way of chemical formula, other bonds are the same as well. Incidentally, R in the drawing represents a stearoyl group.

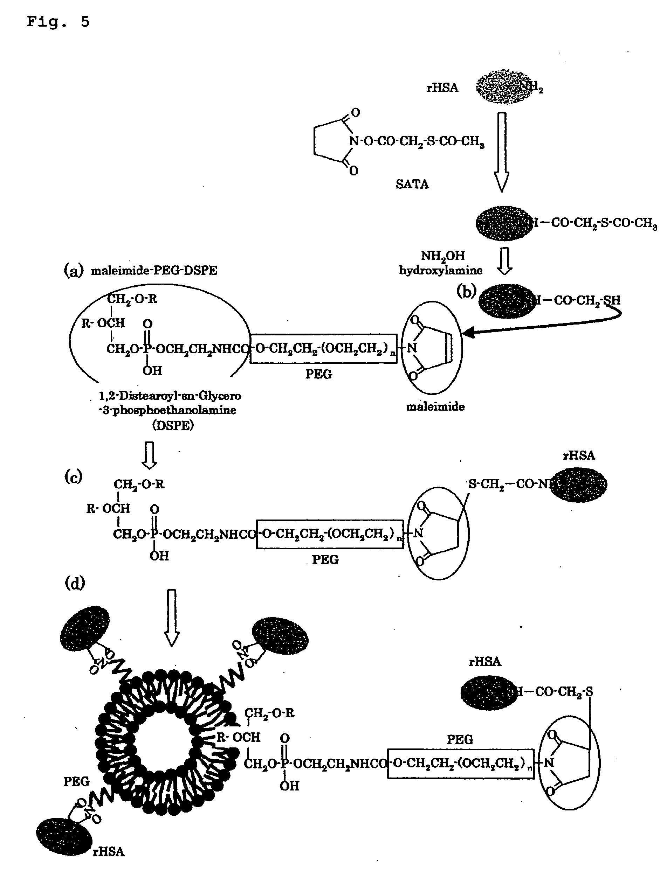

Fig. 5 is a schematic drawing which shows the process for production of a liposome according to Method 2 of Example 2. (a) shows a chemical formula of a maleimide-PEG-modified DSPE. (b) is a schematic drawing of a thiolated rHSA prepared according to the step of Method 2 (2) of Example 2. When the thiolated rHSA shown by the schematic drawing (b) is reacted with the lipid shown by the schematic drawing (a), an rHSA-PEG-DSPE complex shown by the schematic drawing (c) can be prepared. When this rHSA-PEG-DSPE complex is inserted into a previously prepared liposome, there can be obtained the aimed liposome shown by the schematic drawing (d). Here, although only one bond is shown in detail by way of chemical formula among the bond of PEG to rHSA via a maleimide group and bond of the above PEG to the liposome, other bonds are the same as well. Incidentally, R in the drawing represents a stearoyl group.

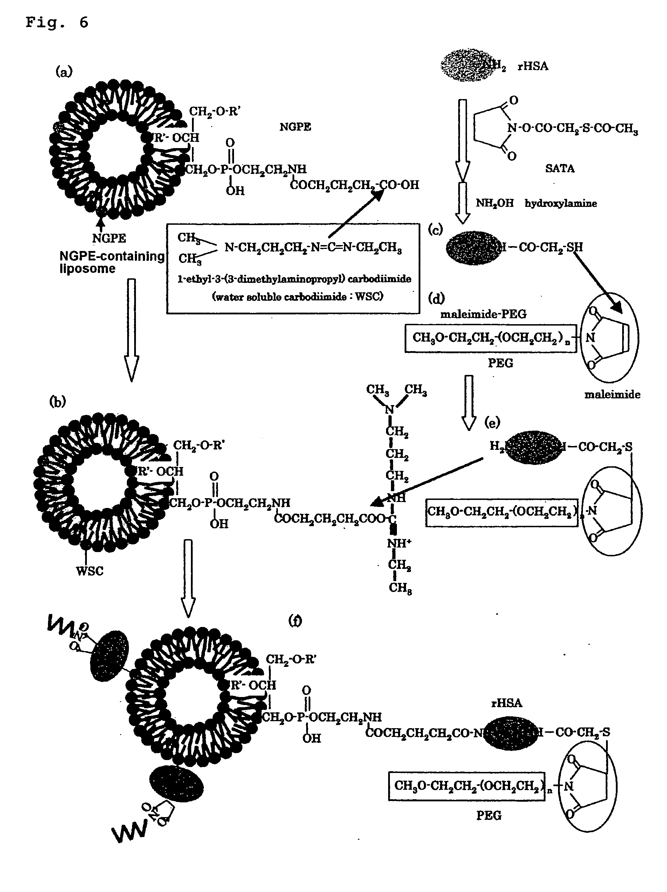

Fig. 6 is a schematic drawing which shows the process for production of a liposome according to Method 1 of Example 3. (a) is a schematic drawing of a PEG-modified liposome containing NGPE. R' in the drawing shows an oleoyl group. Only one of NGPEs in such liposome is shown by the chemical formula. In the step (4) in Method 1 of Example 3, WSC is bonded to the carboxyl group shown by an arrow in NGPE, and then there can be prepared a liposome shown by the schematic drawing (b). In (b), although only one bond among the bonds of NGPE to WSC is shown in detail by way of chemical formula, other bonds are the same as well. (c) is a schematic drawing of a thiolated rHSA prepared according to Method 1(2) of Example 3. When the thiolated rHSA shown by a schematic drawing (c) is reacted with a maleimide-PEG-modified DSPE shown in a schematic drawing (d), there can be prepared the albumin to which a polyethylene glycol is bonded via a maleimide group shown by a schematic drawing (c). When the liposome shown by a schematic drawing (b) is reacted with albumin shown by a schematic drawing (e), there can be obtained the aimed liposome shown by a schematic drawing (f) where the amino group in rHSA is bonded to the carbonyloxy group shown by an arrow. Here, although only one bond among the bonds of PEG to rHSA via a maleimide group and the bond of the above rHSA to the liposome is shown in detail by way of chemical formula, other bonds are the same as well.

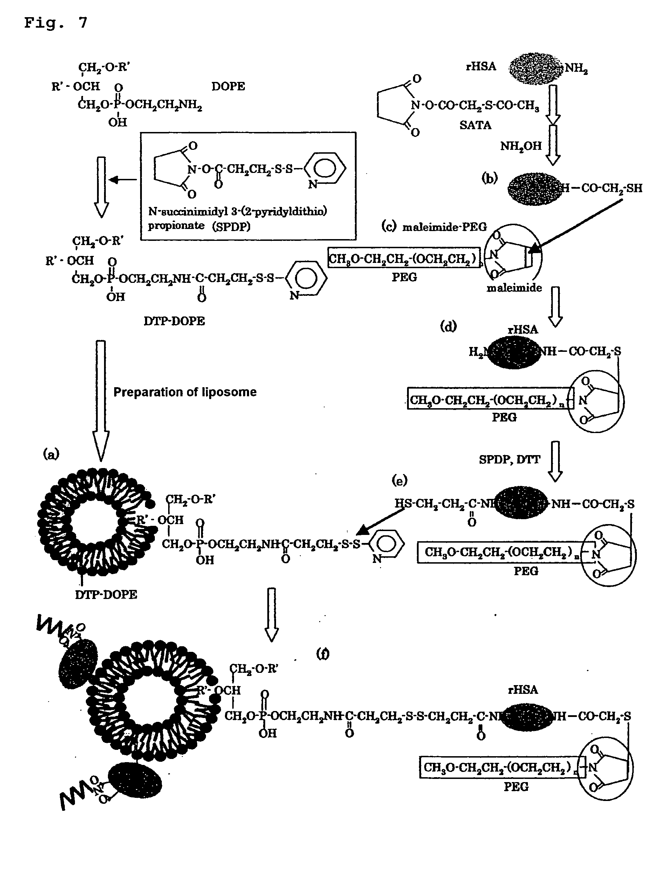

Fig. 7 is a schematic drawing which shows the the process for production of a liposome according to Method 2 of Example 3. In the step (1) of Method 2 of Example 3, DOPE is bonded to SPDP to prepare DTP-DOPE and, in the step (2), the DTP-DOPE as such is used as a constituent lipid to prepare a liposome shown by a schematic drawing (a). R' in the drawing shows an oleoyl group. (b) is a schematic drawing of a thiolated rHSA prepared in the step (3), Method 1 of Example 3. When the thiolated rHSA shown by a schematic drawing (b) is reacted with a maleimide-PEG-modified DSPE shown by a schematic drawing (c), there can be prepared albumin to which a polyethylene glycol is bonded via a maleimide group shown by a schematic drawing (d). When an amino group of the above albumin is converted into a mercapto group in the step (5), there can be prepared albumin shown by a schematic drawing (e). When a liposome shown by a schematic drawing (a) is reacted with albumin shown by a schematic drawing (e), there can be obtained the aimed liposome shown by a schematic drawing (f) wherein the mercapto group is bonded to the dithio group indicated by an arrow.

### Best Mode for Carrying Out the Invention

"Liposome" usually means a liposome composed of a lipid assembled in the form of membranes and an inner aqueous phase and inner aqueous phase (refer to D. D. Lasic, "Liposomes: From Basic to Applications", Elsevier Science Publishers, pp. 1-171 (1933)) and, in the present invention, it means microvesicles as a whole where lipid is aggregated regardless of the fact whether the inner aqueous phase is contained or not. There is also no particular limitation for the structure of the liposome of the present invention and it may be either multilayered liposome or a monolayer liposome.

Although there is no particular limitation for the size of the liposome of the present invention, its volume-average vesicle size is about 10 to 5,000 nm or, preferably, about 50 to 500 nm. The volume-average vesicle size of liposome can be determined on the principle of a dynamic light scattering method, etc. (refer to D. D. Lasic, "Liposomes: From Basic to Applications", Elsevier Science Publishers, pp. 1-171 (1933)).

There is also no particular limitation for the lipid constituting the liposome of the present invention and it may be any one of known lipids. Examples of the lipids as such are phospholipids, glycolipids, fatty acids, dialkyl dimethylammonium amphiphiles, polyglycerol alkyl ethers, polyoxyethylene alkyl ethers, etc. (Liposome Technology, 2nd edition, vol. 1, 141, 1993), alkyl glycosides, alkyl methylglucamides, alkyl sucrose esters, dialkyl polyoxyethylene ethers, dialkyl polyglycerol ethers, etc. (Liposome Technology, 2nd edition, vol. 1, 141, 1993), amphipathic block copolymers, etc. such as polyoxyethylene-polylactic acid (Japanese Patent Laid-Open No. 06/508,831), long-chain alkylamines (tetradecylamine, hexadecylamine, stearylamine, etc.) or long-chain fatty acid hydrazides (myristic acid hydrazide, palmitic acid hydrazide or stearic acid hydrazide, etc.), etc.

Examples of the above phospholipid are natural or synthetic ones such as phosphatidylcholine (soybean phosphatidylcholine, egg yolk phosphatidylcholine, dilauroyl phosphatidylcholine, dimyristoyl phosphatidylcholine, dipalmitoyl phosphatidylcholine or distearoyl phosphatidylcholine, etc.), phosphatidylethanolamine (dilauroyl phosphatidylethanolamine, dimyristoyl phosphatidylethanolamine, dipalmitoyl phosphatidylethanolamine or distearoyl phosphatidylethanolamine, etc.), phosphatidylserine (dilauroyl phosphatidylserine, dimyristoyl phosphatidylserine, dipalmitoyl phosphatidylserine or distearoyl phosphatidylserine, etc.), phosphatidic acid, phosphatidylglycerol (dilauroyl phosphatidylglycerol, dimyristoyl phosphatidylglycerol, dipalmitoyl phosphatidylglycerol or distearoyl phosphatidylglycerol, etc.), phosphatidylinositol (dilauroyl phosphatidylinositol, dimyristoyl phosphatidylinositol, dipalmitoyl phosphatidylinositol or distearoyl phosphatidylinositol, etc.), lysophosphatidylcholine, sphingomyelin, egg yolk lecithin, soybean lecithin or hydrogenated phospholipid, etc.

Examples of the above glycolipid are glyceroglycolipid, sphingoglycolipid and sterols, etc. Examples of the above glyceroglycolipid are digalactosyl diglycerides (digalactosyl dilauroyl diglyceride, digalactosyl dimyristoyl glyceride, digalactosyl dipalmitoyl glyceride or digalactosyl distearoyl glyceride, etc.) or galactosyl diglycerides (galactosyl dilauroyl glyceride, galactosyl dimyristoyl glyceride, galactosyl dipalmitoyl glyceride or galactosyl distearoyl glyceride, etc.) and the like. Examples of the above sphingoglycolipid are galactosyl cerebroside, lactosyl cerebroside or ganglioside, etc. Examples of the above sterol are cholesterol, cholesterol hemisuccinate, 3β-[N-(N',N'-dimethylaminoethane)carbamoyl]cholesterol, ergosterol or lanosterol, etc.

In the present invention, the lipid as such may be used either solely or jointly by combining two or more thereof.

Although there is no particular limitation for the polyalkylene glycol used in the present invention, that where an alkylene chain having 1 to 6 carbon atom(s) is preferred. The alkylene chain may be substituted with substituent(s) which do/does not affect the present invention such as hydroxy group, carboxyl group, amino group and alkoxy group, etc. To be more specific, polyethylene glycol (PEG), polypropylene glycol, etc. may be used and the use of polyethylene glycol is particularly preferred. Although there is no particular limitation for molecular weight of the polyalkylene glycol, there may be employed a polyalkylene glycol having a molecular weight of about 200 to 4,000,000, preferably about 1,000 to 50,000. When a polyethylene glycol is used, those having the above-mentioned molecular weight are particularly preferred.

Although there is no particular limitation for the amount of the polyalkylene glycols, about 0.5 to 30 molar % to the total lipid amount constituting the liposome is preferred.

There is no particular limitation for albumin used in the present invention and its examples are animal albumin such as egg albumin, serum albumin, milk albumin or muscle albumin (myogen) and plant albumin such as leucosin, legumelin or ricin. Among them, it is preferred in the present invention to use serum albumin of the same animal which is an object to be administered. Albumin used in the present invention may also be the albumin prepared by a recombinant gene technique. The above albumin may have the same amino acid sequence as that of a wild type albumin or may be a mutant type albumin in which one or plural or, preferably, one to several amino acid(s) is/are deleted, substituted or added provided that it is not against the object of the present invention. Such an albumin may be easily prepared by a known art. In the present invention, it is preferred to use a genetically recombined albumin since there is no risk of infection.

In the present invention, although there is no particular limitation for the amount of albumin, it is preferred to be about 0.0001 to 10 molar % to the total amount of lipid constituting liposome.

Liposome of the present invention may be in any structure so far as it contains the above-mentioned polyalkylene glycol and albumin. Mode and position of the bond of polyalkylene glycol to albumin may be in any mode and at any position for such bond formation. It is however preferred that liposome of the present invention has a polyalkylene glycol and albumin on its surface. In addition, although a polyalkylene glycol and albumin may be bonded to the liposome in any form such as adsorption, electric bond, physical bond (e.g., van der Waals force) and chemical bond, it is preferred to be bonded by means of chemical bond.

With regard to a preferred embodiment of the liposome of the present invention, case (a) where each of polyalkylene glycol and albumin is bonded to a liposome may be exemplified. Also, case (b) where a liposome and albumin are bonded via a polyalkylene glycol may also be exemplified. Thus, it is a case where a liposome is bonded to a polyalkylene glycol and, at the site which is different from the above bonding site, albumin is bonded to polyalkylene glycol. There is still another case (c) where a liposome and a polyalkylene glycol are bonded via albumin. Thus, it is a case where a liposome is bonded to albumin and, at the site which is different from the above bonding site, a polyalkylene glycol is bonded to albumin. In the present invention, the liposomes of the above-mentioned embodiments of (a) to (c) may be present in a mixed state.

The liposome of the present invention may be produced using a known techniques. Preferred process for the production of the liposome according to the present invention will be illustrated as hereunder for each of the above-mentioned three embodiments.

(a) In case where a polyalkylene glycol and albumin are bonded to a liposome, examples of the process for the production of liposome according to the present invention includes (i) a process where albumin is bonded to a liposome to which a polyalkylene glycol is bonded, (ii) a process where a polyalkylene glycol is bonded to a liposome to which albumin is bonded and (iii) a process where a liposome is produced using a lipid to which a polyalkylene glycol is bonded and a lipid to which albumin is bonded.

In the above process (i), a liposome to which a polyalkylene glycol is bonded can be easily produced using a known process. An example is a process where a liposome is produced using a lipid to which a polyalkylene glycol is bonded. Examples of the above "lipid to which a polyalkylene glycol is bonded" are polyalkylene glycol-modified phospholipids, polyalkylene glycol alkyl ethers, polyalkylene glycol castor oil derivatives and polyalkylene glycol sorbitan fatty acid esters. With regard to the "polyalkylene glycol" moiety of such lipid, a polyethylene glycol is preferred. With regard to a "lipid to which a polyalkylene glycol is bonded", a polyethylene glycol-modified phospholipid is preferred, and that in which the phospholipid is phosphatidylethanolamine is more preferred.

To be more specific, for example, there are exemplified PEG-DSPE [1,2-distearoyl-*sn*-glycero-3-phosphatidylethanolamine-N-(polyethylene glycol)],
N-monomethoxypolyethylene glycol succinyl phosphatidylethanolamine represented by the following formula (11):

   CH₃O-(CH₂CH₂O)ₙ-CO-CH₂CH₂-CO-NH-PE (11)

   (wherein n is an integer of 5 to 100,000, preferably an integer of 10 to 1,200, and -NH-PE is a phosphatidylamino group),
N-monomethoxypolyethylene glycol (2-chloro-1,3,5-triazine-4,6-diyl)succinyl phosphatidylethanolamine represented by the following formula (12): 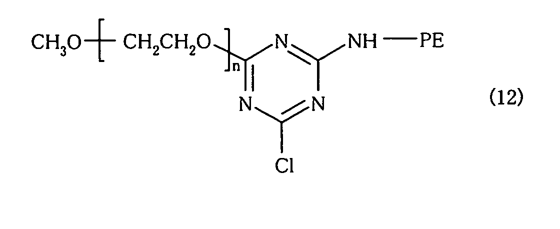 (wherein n and -NH-PE have each the same meanings as defined above),
N-monomethoxypolyethylene glycol carbonyl phosphatidylethanolamine represented by the following formula (13):

   CH₃O-(CH₂CH₂O)ₙ₋₁-CO-NH-PE (13)

   (in the formula, n and -NH-PE have the same meaning as defined above) and N-monomethoxypolyethylene glycol ethylene phosphatidylethanolamine represented by the following formula (14):

   CH₃O-(CH₂CH₂O)ₙ-CH₂CH₂-NH-PE (14)

   (wherein n and -NH-PE have each the same meaning as defined above).

The "lipid to which a polyalkylene glycol is bonded" as above can be easily produced using a known method or a commercially available product may be used. With regard to a process for the production of a liposome using such lipid as a constituent lipid, there is no particular limitation but a known process may be used. For example, a liposome may be produced using the above-mentioned lipid and an aqueous phase by means of a thin film method, a reversed phase evaporation method, an ethanol injection method, an ether injection method, a dehydration-rehydration method, etc. and a volume-average vesicle size may be adjusted by means of an ultrasonic wave irradiation method, an ultrasonic wave irradiation after freezing/thawing, an extrusion method, a French press method, a homogenization method, etc. (refer to D. D. Lasic, "Liposomes: From Basic to Applications", Elsevier Science Publishers, pp. 1-171 (1933)). Here, "aqueous phase" means an aqueous solution constituting the inner area of liposome and, although there is no particular limitation therefor so far as it is commonly used in the related technical field, preferred one is an aqueous sodium chloride solution, a buffer such as phosphate buffer or acetate buffer, an aqueous saccharide solution such as aqueous glucose solution or aqueous trehalose solution or a mixture thereof. In order to keep the structure of liposome stable when administered into the living body, it is usually preferred that an aqueous phase used for the production of a liposome is nearly isotonic to outside of liposome or, in other words, to body fluid and that osmotic pressures applied to inside and outside of the liposome are a little.

In order to bond albumin to the resulting liposome to which a polyalkylene glycol is bonded, such bonding can be easily carried out using a known means such as a sulfhydryl-maleimide coupling technique (Derksen, J. T. P. and Scherphof, G. L. (1985), *Biochem. Biophys. Acta,* 814, p. 151-155). Particularly, a process where the above liposome and albumin are bonded via a reactive intervening group can be advantageously adopted. There is no particular limitation for the reactive intervening group, and it may be a group which is known in the related technical field.

With regard to preferred embodiments, the following methods may be exemplified. Thus, in the production of a polyalkylene glycol-bonded liposome, a lipid having a reactive intervening group is used as a constituent lipid in addition to the polyalkylene glycol-bonded lipid. With regard to a lipid having a reactive intervening group, 1,2-dioleyl-*sn*-glycero-3-phosphoethanolamine-N-(glutaryl) (hereinafter, it will be abbreviated as "NGPE") is preferred. Polyalkylene glycol-bonded liposome is produced as mentioned above using such constituent lipids and then albumin is bonded thereto via the reactive intervening group in the above liposome. When NGPE is used, it is preferred that the amino group of albumin is bonded to the terminal carboxyl group of NGPE. At that time, it is also possible that a functional group for enhancing the reactivity of the reactive intervening group of the above liposome is bonded previously and then albumin may be bonded as such that it is substituted for the functional group. For example, when NGPE is used, carbodiimide group is previously bonded to NGPE using a water-soluble carbodiimide and then albumin is bonded to NGPE as such that it is substituted for the carbodiimide group.

Another preferred embodiment is a process wherein a lipid (hereinafter, it will be abbreviated as "DTP-DOPE") in which 3-(2-pyridylthio) propionate (hereinafter, it will be abbreviated as "DTP") is bonded to the amino group of 1,2-dioleyl-*sn*-glycero-3-phosphoethanolamine (hereinafter, it will be abbreviated as "DOPE") is used as a lipid having a reactive intervening group. To be more specific, a polyalkylene glycol-bonded liposome is produced as mentioned above using DTP-DOPE as a constituent lipid in addition to a lipid to which a polyalkylene glycol is bonded. In the meanwhile, a mercapto group is introduced into albumin. There is no particular limitation for a method of introduction of a mercapto group but a known method may be used. Preferably, DTP is introduced into albumin and then reaction with dithiothreitol is conducted to obtain albumin into which mercapto group is introduced. When the above liposome is reacted with albumin, albumin can be bonded to a polyalkylene glycol-bonded liposome.

Processes mentioned in the above (ii) and (iii) may be easily carried out according to the description mentioned above.

(b) In case liposome and albumin are bonded via a polyalkylene glycol, examples of the process for the production of the liposome according to the present invention are (i) a process where a liposome to which a polyalkylene glycol is bonded is produced and the albumin is bonded to the polyalkylene glycol of the above liposome and (ii) a process where a polyalkylene glycol to which albumin is bonded is bonded to a liposome at the site which is different from the site to which albumin is bonded.

In the above-mentioned process (i), a process for the production of a polyalkylene glycol-bonded liposome is the same as that mentioned above. For bonding albumin to a polyalkylene glycol of the liposome, a known means may be used. Particularly a means where a polyalkylene glycol is bonded to albumin via a reactive intervening group can be adopted. There is no particular limitation for the reactive intervening group but any group which is known in the related art may be used and a maleimide group may be exemplified as an appropriate example.

To be more specific, the following process may be mentioned. Thus, in a lipid to which a polyalkylene glycol is bonded, a reactive functional group is bonded to a polyalkylene glycol. For example, it is preferred that a maleimide group is bonded to the hydroxy group of the polyalkylene glycol. The resulting lipid is used to produce a liposome by the same manner as mentioned above. When the resulting liposome is reacted with albumin, albumin is bonded via the reactive functional group of polyalkylene glycol bonding to a liposome whereupon the objective liposome is obtained. At that time, known treatment such as that a substituent group corresponding to the reaction functional group is introduced into albumin so that the reactive functional group is apt to be bonded to albumin may be conducted. When the reactive functional group is a maleimide group, it is preferred that a mercapto group is previously introduced into albumin. There is no particular limitation for a method of introduction of a mercapto group but a known method may be used. To be more specific, albumin is reacted with acetyl thioacetate so that acetyl thioacetate is bonded to the amino group of albumin and then acetyl group is removed, thereby to introduce an mercapto group into albumin.

In the above-mentioned process (ii), there is no particular limitation for a method wherein a polyalkylene glycol and albumin are bonded but a known means may be used, and it is preferred to carry out the bonding via a reactive intervening group. There is no particular limitation for a reactive intervening group but a known group in the related technical field may be used, and a maleimide group may be exemplified as an appropriate example. After that, a liposome is bonded to the resulting albumin-bonded polyalkylene glycol at a site different from the bonding site to albumin. Again, there is no particular limitation for the method therefor but a known means may be used. A preferred example is that a polyalkylene glycol to which lipid is previously bonded is used as a polyalkylene glycol and the albumin-polyalkylene glycol-lipid complex produced in the above step is inserted into a liposome.

To be more specific, a polyalkylene glycol is bonded to a lipid as mentioned above. After that, a reactive functional group is bonded to a polyalkylene glycol of the resulting lipid. For example, it is preferred that maleimide group is bonded to hydroxyl group of polyalkylene glycol. After that, albumin is bonded via a reactive functional group to a polyalkylene glycol of the resulting lipid. At that time, albumin may be subjected to a known treatment such as introduction of a substituent corresponding to the above reactive functional group into albumin so that the above reactive functional group is apt to be bonded to albumin. When the above reaction functional group is a maleimide group, it is preferred that a mercapto group is previously introduced into albumin. There is no particular limitation for a method of introduction of a mercapto group but a known method may be used. To be more specific, albumin is reacted with acetyl thioacetate so that acetyl thioacetate is bonded to the amino group of albumin and then acetyl group is removed, thereby to produce an albumin to which a mercapto group is introduced. On the other hand, a liposome is produced by a known method and the resulting albumin-polyalkylene glycol-lipid complex is inserted into a liposome to produce the objective liposome.

(c) In case where a liposome and a polyalkylene glycol are bonded via albumin, examples of the process for the production of liposomes according to the present invention are (i) a process where a liposome is bonded to albumin to which a polyalkylene glycol is bonded at a site different from the bonding site of polyalkylene glycol and (ii) a process where a liposome to which albumin is bonded is prepared and then a polyalkylene glycol is bonded to albumin of the above liposome.

In the above-mentioned process (i), there is no particular limitation for a method for bonding of polyalkylene glycol and albumin, but a known means may be used, and it is preferred to perform such bonding via a reactive intervening group. There is no particular limitation for the reactive intervening group, but a group known in the related field may be used, and a maleimide group is an appropriate example. To be more specific, a reactive functional group is bonded to a polyalkylene glycol. For example, it is preferred that a maleimide group is bonded to the hydroxy group of polyalkylene glycol. After that, albumin is bonded via a reactive functional group to the polyalkylene glycol. At that time, albumin may be subjected to a known treatment such as introduction of a substituent corresponding to the above reactive functional group into albumin so that the above reactive functional group is apt to be bonded to albumin. When the above reaction functional group is a maleimide group, it is preferred that a mercapto group is previously introduced into albumin. There is no particular limitation for a method of introduction of a mercapto group but a known method may be used. To be more specific, albumin is reacted with acetyl thioacetate so that acetyl thioacetate is bonded to the amino group of albumin and then acetyl group is removed to produce an albumin into which a mercapto group is introduced.

After that, a liposome is bonded to the resulting albumin to which polyalkylene glycol is bonded. The method therefor is as mentioned above.

In the above-mentioned process (ii), such method for bonding albumin to a liposome is as mentioned above. After that, a polyalkylene glycol is bonded to the above albumin. A method therefor may be also the same as above.

The following intermediates produced in the above-mentioned production processes are novel substances.
A compound represented by the formula (3):

   (Alb-NH)-CO-CH₂-CH₂-SH (3)

   (wherein Alb-NH is a group formed by removal of one hydrogen atom from the amino group of albumin molecule represented by Alb-NH₂),
a compound represented by the formula (5):

   (Alb-NH)-CO-CH₂-SH (5)

   (wherein Alb-NH has the same meaning as defined above),
   a compound represented by the formula (6): 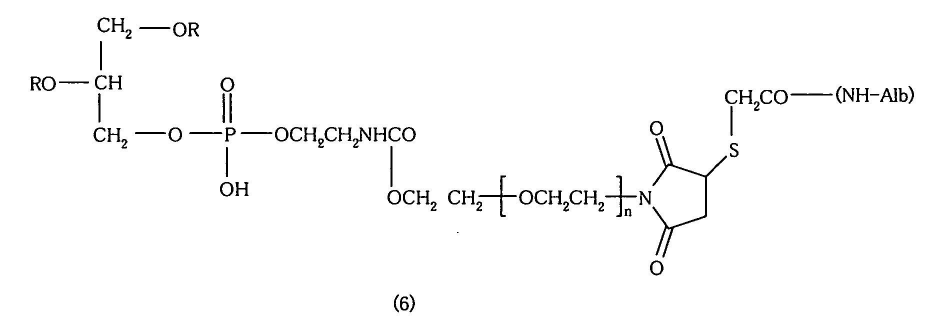 (wherein Alb-NH has the same meaning as defined above; n is an integer of 5 to 100,000, preferably an integer of 10 to 1,200; R is an acyl group derived from a saturated or unsaturated fatty acid having 2 to 35 carbon atoms. More preferably, the above fatty acid has 6 to 18 carbon atoms and, most preferably, 8 to 16 carbon atoms. Specific examples of such fatty acid are octanoic acid (preferably, caprylic acid), decanoic acid (preferably, capric acid), dodecanoic acid (preferably, lauric acid), hexadecanoic acid (preferably, palmitic acid), octadecanoic acid (preferably, stearic acid) and monoenoic or polyenoic fatty acid thereof (preferably, oleic acid). Specific examples of an acyl group derived from the fatty acid as such are octanoyl group (preferably, capryloyl group), decanoyl group (preferably, caprinoyl group), dodecanoyl group (preferably, lauroyl group), hexadecanoyl group (preferably, palmitoyl group) and octadecanoyl group (preferably, stearoyl group) and there may be one or more double bond(s) therein (such as oleoyl group)),
a compound represented by the formula (7): 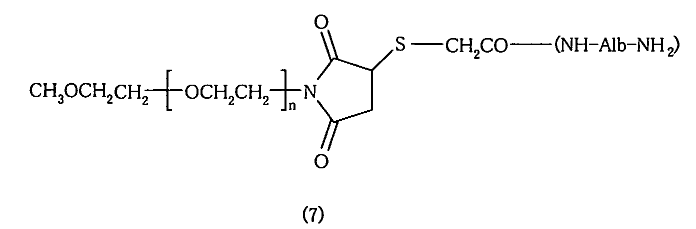 (wherein -NH-Alb-NH₂ is a group formed by removal of one hydrogen atom from one amino group of albumin molecule represented by H₂N-Alb-NH₂ and n has the same meaning as defiined above), and
a compound represented by the formula (8): 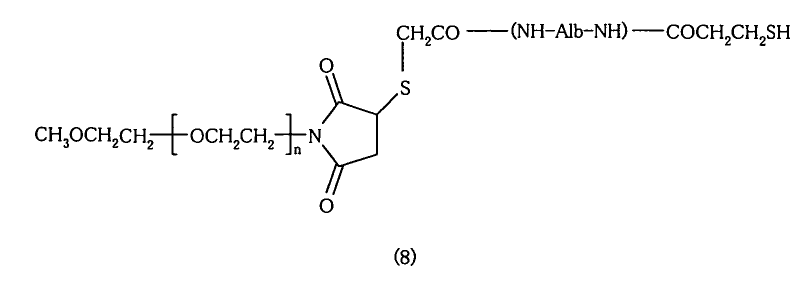 (wherein -NH-Alb-NH- is a group formed by removal of each one hydrogen from the two amino groups of albumin molecule represented by the formula H₂N-Alb-NH₂, and n has the same meaning as defined above).

Preferably, the liposome of the present invention is used in the form of being carried with a physiologically active ingredient. There is no particular limitation for the manner of carrying the physiologically active ingredient. For example, the physiologically active ingredient may be included in the liposome or may be either adsorbed on or bonded to the surface of liposome. Further, the physiologically active ingredient may be either adsorbed on or bonded to the albumin or polyalkylene glycol.

There is no particular limitation for the physiologically active ingredient so far as it is a compound or a composition of matter which is able to be administered to animals or, preferably, to human. For example, the physiologically active ingredient includes a compound or a composition which exerts a physiological activity and is effective for prevention or treatment of diseases, a compound or a composition used for diagnosis (e.g., a contrast medium), and a gene useful for gene therapy. Specific examples of the physiologically active ingredient are antiviral agents such as acyclovir, zidovudine and interferon; antibacterial agents such as aminoglycoside, cephalosporin and tetracycline; antimycotic agents such as polyene antibiotic, imidazoles and triazoles; antimetablites such as folic acid, purine and pyrimidine analogs; antitumor agents such as anthracycline antibiotic and plant alkaloid; sterols such as cholesterol; carbohydrates such as saccharide and starch; amino acids, peptides and proteins such as cell receptor protein, immunoglobulin, enzyme, hormone, neurotransmitter and glycoprotein; dyes; radioactive labeling agents such as radioisotope and radioisotope-labeled compound; radiopacity agents; fluorescent compounds; mydriatic compounds; bronchial dilators; and local anesthetics.

In the present invention, it is particularly preferred to use an antitumor agent as a physiologically active ingredient. Although there is no particular limitation for the antitumor agent, its examples are alkylating agents, various metabolic antagonists, antitumor antibiotics, other antitumor agents, antitumor plant components, BRM (biological response modifies), angiogenesis inhibitors, cell adhesion inhibitors, matrix-metalloprotease inhibitors and hormones.

To be more specific, examples of alkylating agent are those such as nitrogen mustard, nitrogen mustard N-oxide and chlorambucil; aziridine-type alkylating agents such as carboquone and thiotepa; epoxide-type alkylating agents such as dibromomannitol and dibromodulcitol; nitrosourea-type alkylating agents such as carmustine, lomustine, semustine, nimustine hydrochloride, streptozocin, chlorozotocin and ranimustine; busulfan; improsulfan tosylate; and dacarbazine. Examples of various metabolic antagonists are purine metabolic antagonists such as 6-mercaptopurine, 6-thioguanine and thioinosine; pyrimidine metabolic antagonists such as fluorouracil, tegafur, tegafur uracil, carmofur, doxifluridine, broxiuridine, cytarabine and enocitabine; folic acid metabolic antagonists such as methotrexate and trimetrexate; and salts and complexes thereof.

Examples of antitumor antibiotics are anthracycline type antibiotic antitumor agents such as mitomycin A, bleomycin, peplomycin, daunorubicin, aclarubicin, doxorubicin, pirarubicin, THP-adriamycin, 4'-epidoxorubicin and epirubicin; chromomycin A₃; actinomycin D; and salts or complexes thereof. Examples of other antitumor agents are cisplatin, carboplatin, tamoxifen, camptothecin, ifosfamide, cyclophosphamide, melfalan, L-asparaginase, aceglatone, sizofiran, picibanil, ubenimex, Krestin and salts or complexes thereof. Other examples are procarbazine, pipobroman, neocarzinostatin and hydroxyurea.

Examples of antitumor plant component are vinca alkaloids such as vindesine, vincristine and vinblastine; epipodophyllotoxins such as etoposide and teniposide; and salt or complex thereof. Examples of BRM are tumor necrosis factor, indomethacin and salts or complexes thereof. Examples of angiogenesis inhibitor are fumagillol derivative and salts or complexes thereof. Examples of cell adhesion inhibitor are a substance having an RGD sequence (Arg-Gly-Asp) and salts or complexes thereof. Examples of matrix metalloprotease inhibitor are marimastat, batimastat and salts or complexes thereof. Examples of hormone are hydrocortisone, dexamethasone, methyl prednisolone, prednisolone, prasterone, betamethasone, triamcinolone, oxymetholone, nandrolone, metenolone, fosfestrol, ethynylestradiol, chlormadinone, medroxyprogesterone and salts or complexes thereof.

Although the pharmaceutical composition of the present invention may comprise only the liposome of the present invention in which the above physiologically active ingredient is carried, it is usually prepared by mixing the liposome with a pharmacologically acceptable carrier by a known method per se [a method which has been commonly used in the field of pharmaceutical preparations such as a method mentioned in the Japanese Pharmacopoeia (e.g., 13th revision)]. With regard to the pharmaceutically acceptable carrier, various kinds of organic or inorganic carriers which have been common as a material for pharmaceutical preparations may be used. Examples of such carriers are excipients, lubricants, binders and disintegrating agents in solid preparations; and solvents, solubilizers, suspending agents, isotonic agents, buffers and soothing agents. If necessary, it is also possible to use additives for pharmaceutical preparations such as surfactants, foaming agents, dyes, acidifying agents, antiseptics, antioxidants, coloring agents, sweeteners and corrigent.

With regard to the pharmaceutically acceptable carrier, more specific examples are excipients of inorganic salts such as potassium citrate and calcium phosphate; lubricants such as magnesium stearate, calcium stearate, light silicic acid anhydride and aqueous silicon dioxide; binders such as hydroxypropyl cellulose, hydroxypropylmethyl cellulose, α-starch, polyvinyl alcohol, polyvinylpyrrolidone, gum arabic, gelatin and pullulan; and disintegrating agents such as cellulose (e.g., hydroxypropyl cellulose with low substitution degree and crystalline cellulose), various starches or starch derivatives (e.g., corn starch, partial α-starch and hydroxypropyl starch), crospovidone and bentonite.

Other examples are solvents such as salt solution, glucose solution and a mixture of salt solution and glucose solution; solubilizers such as dextran, polyvinylpyrrolidone, sodium benzoate, ethylenediamine, salicylamide, nicotinamide and polyoxyethylene hydrogenated castor oil; buffers such as borate buffer, phosphate buffer, citrate buffer, tartrate buffer and acetate buffer; albumin; polyvalent alcohol such as glycerol and propylene glycol; and soothing agents such as lidocaine hydrochloride and benzyl alcohol.

Further examples are surfactants such as sorbitan fatty acid ester, polyoxyethylene fatty acid ester, phospholipid, glycerol fatty acid ester, polyethylene glycol fatty acid ester, polyoxyethylene hydrogenated castor oil, polyoxyethylene alkyl ether and sucrose fatty acid ester; foaming agents such as sodium hydrogen carbonate, sodium carbonate and calcium carbonate; acidifying agent such as citric acid, tartaric acid and malic acid; dyes such as iron sesquioxide, yellow iron sesquioxide and tar dye; flavors such as lemon, lemon lime, orange, pineapple, mint and menthol; sweeteners such as saccharin sodium, glycyrrhizin dipotassium, aspartame, stevia and thaumatin; and corrigents such as citric acid, sodium citrate, succinic acid, tartaric acid, fumaric acid and glutamic acid.

Furthermore, with regard to a stabilizer, saccharides and sodium sulfite are exemplified. Examples of the saccharide are monosaccharide such as glucose, fructose, xylitol, fucose and galactose; disaccharides such as maltose, sucrose, lactose, lactulose and melibiose; oligosaccharides such as fructooligosaccharide, galactooligosaccharide and lactooligosaccharide; and polysaccharides such as dextran. Examples of preservative are p-oxybenzoate, benzyl alcohol, chlorocresol, phenethyl alcohol and benzethonium chloride. Examples of chelating agent are sodium edetate and sodium citrate. Examples of antioxidant are sodium sulfite, sodium hydrogen sulfite, sodium ascorbate and sodium thiosulfate.

Dosage form of the drug according to the present invention are oral preparations such as tablet, capsule (including soft capsule, microcapsule and enteric capsule), diluted powder, granule and syrup; and parenteral preparation such as injection preparation (e.g., subcutaneous injection preparation, intravenous injection preparation, intramuscular injection preparation and intraperitoneal injection solution), agents for external application (e.g., nasal preparation, percutaneous preparation and ointment), suppositories (e.g., rectal suppositori and vaginal suppository), pellets, infusion preparations and sustained-release preparations (e.g., sustained-release microcapsule). It is particularly preferred that the drug according to the present invention is in a dosage form of injection preparation.

Although dose of the drug of the present invention is not able to be definitely decided since it varies depending upon the kind of the physiologically active ingredient in the liposome, dosage form of the drug, the kind of diseases to be treated, severity of symptoms and disease to be treated, age, sex or body weight of a patient, administration route, etc., it may be decided by an overall judgment of a medical doctor for the above-mentioned factors.

There is no particular limitation for the administration route for the drug of the present invention and, depending upon the dosage form according to the present invention as mentioned above, it may be administered orally or parenterally. For example, when the drug of the present invention is an injection preparation, a medically appropriate administration form such as intravenous injection, subcutaneous injection, intracutaneous injection, intramuscular injection or intraperitoneal injection may be exemplified.

The drug according to the present invention is able to prevent or treat various diseases depending upon the kind of the physiologically active substance carried on the liposome of the present invention. For example, when the physiologically active substance is an antitumor agent, the drug according to the present invention is useful as prevention or treatment of tumor such as colorectal cancer, brain tumor, head and neck cancer, breast cancer, lung cancer, esophageal cancer, stomach cancer, liver cancer, gallbladder cancer, bile duct cancer, pancreatic cancer, islet cell cancer, choriocarcinoma, colon cancer, renal cell cancer, adrenocortical cancer, bladder cancer, testicular cancer, prostatic cancer, testicular tumor, ovarian cancer, uterine cancer, choriocarcinoma, thyroid gland cancer, malignant carcinoid tumor, skin cancer, malignant melanoma, osteosarcoma, soft tissue sarcoma, neuroblastoma, Wilms' tumor, retinoblastoma, melanoma and squamous cell carcinoma.

### Examples

As shown below, the present invention will be illustrated in detail by way of Examples although it goes without saying that the present invention is not limited to the following Examples. rHSA used in the following Examples was purchased from Bifa Co. Definitions for the abbreviations mentioned in the Examples are as follows.
- rHSA:: recombinant human serum albumin
- PEG:: polyethylene glycol
- DSPE:: 1,2-distearoyl-*sn*-glycero-3-phosphoethanolamine
- NGPE:: 1,2-dioleoyl-*sn*-glycero-3-phosphoethanolamine-N-(glutaryl)
- NHS:: N-hydroxysuccimide
- WSC:: water soluble carbodiimide
- SPDP:: N-succinimidyl 3-(2-pyridyldithio)propionate
- DTP:: 3-(2-pyridyldithio) propionate
- DOPE:: 1,2-dioleoyl-*sn*-glycero-3-phosphoethanolamine
- DTT:: dithiothreitol
- SATA:: N-succinimidyl-S-acetylthioacetate
- HEPES:: N-2-hydroxyethylpiperazine-N'-2-ethanesulfonic acid
- EDTA:: ethylenediamine tetraacetic acid sodium salt
- PBS:: phosphate buffer of pH 7.4 (comprising sodium chloride, potassium chloride, potassium dihydrogen phosphate and disodium hydrogen phosphate)

### Example 1

### Manufacture of a liposome wherein PEG and rHSA are bonded to the surface thereof

### Method 1: Production using WSC

### (1) Production of PEG-modified liposome containing NGPE

About 8 mL of chloroform solution in which lipid was dissolved (total lipid: 100 µmol; dissolved in a molar ratio of egg yolk lecithin : cholesterol : NGPE : PEG-bonded DSPE (manufactured by Shearwater Co.) = 59 : 20 : 10 : 1) was added to an eggplant type flask. After that, 2,000,000 dpm of [³H (tritium)] cholesteryl hexadecyl ether (manufactured by Japan Radioisotope Association) was added. Chloroform was added so as to make a total volume of 10 mL, and the solvent was evaporated *in vacuo* using a rotary evaporator under a nitrogen atmosphere followed by drying for one night. To the resulting thin film of lipid was added about 2 mL of PBS, and the mixture was mixed with stirring for 15 minutes or longer by heating at 55°C so that the thin film of lipid was suspended. Particle size of the suspension was made uniform using an extruder equipped with a polycarbonate membrane of 200 nm.

### (2) Production of rHSA-PEG-modified liposome

To the above PEG-modified liposome were added 100 µmol of NHS and 10 µmol of WSC, and the mixture was stirred for 15 minutes so that NGPE and WSC were bonded thereto, after which time 500 µmol of 2-mercaptoethanol was added. In order to remove NHS and WSC which were not bonded to liposome, substances of low molecular weight were separated from liposome fraction by means of a gel filtration to recover a liposome fraction. To the liposome fraction was immediately added 1 µmol of rHSA, and the mixture was shaken overnight so that rHSA was bonded to liposome. For the purpose of removing unreacted rHSA, a gel filtration was carried out to separate into an rHSA-bonded liposome fraction and a fraction of single rHSA, resulting in recovery of an rHSA-bonded liposome fraction (10 mL).

### Method 2: Production using SPDP

### (1) Production of DTP-DOPE

SPDP (12 µmol) was added to a solution of DOPE in chloroform (10 µmol), and the solution was stirred. To the reaction solution was added about 2 mL of PBS, and the mixture was vigorously stirred for 5 minutes and then centrifuged at 3,000 rpm for 10 minutes. After removal of the PBS layer, pure water was added and the mixture was vigorously shaken for 3 minutes and centrifuged to remove an aqueous layer again. Washing with pure water as such was conducted once again. A white semi-solid remaining in the lower layer was subjected to solvent evaporation and dried using an evaporator. The residue was added with 1.0 mL of chloroform and redissolved to produce DTP-DOPE.

### (2) Production of PEG-modified liposome containing DTP-DOPE

About 8 mL of chloroform solution in which lipid was dissolved (total lipid: 100 µmol; dissolved in a molar ratio of egg yolk lecithin : cholesterol : PEG-bonded DSPE (manufactured by Shearwater Co.) = 59 : 30 : 1) was added to an eggplant type flask. After that, 10 µmol of DTP-DOPE was added and 2,000,000 dpm of [³H (tritium)] cholesteryl hexadecyl ether (manufactured by Japan Radioisotope Association) was added. Chloroform was added so as to make a total volume of 10 mL, and the solvent was then evaporated *in vacuo* using a rotary evaporator under a nitrogen atmosphere, and dried overnight. To the resulting thin film of lipid was added about 2 mL of PBS, and the mixture was mixed with stirring for 15 minutes or longer by heating at 55°C so that the thin film of lipid was suspended. Particle size of the suspension was made uniform using an extruder equipped with a polycarbonate membrane of 200 nm.

### (3) Production of thiolated rHSA

SPDP (20 µmol) was added to 1 µmol of an aqueous solution of rHSA, followed by stirring to produce DTP-rHSA. In order to remove the unreacted SPDP, a gel filtration was conducted to collect a PD-rHSA fraction. DTT was added to the DTP-rHSA to make a final concentration of 50 mM, and the mixture was stirred for 20 minutes so that rHSA was thiolated. For the purpose of removing the unreacted DTT, gel filtration was conducted to collect a thiolated rHSA fraction.

### (4) Reaction of DTP-DOPE-containing liposome with thiolated rHSA

To PEG-modified liposome containing DTP-DOPE was added a solution of thiolated rHSA, and the mixture was stirred at room temperature for 24 hours or longer. After that, the reaction solution was subjected to a gel filtration to separate into liposome and unreacted rHSA, and a liposome fraction was recovered to give a rHSA-PEG-modified liposome.

### Example 2

### Method for the production of liposome wherein PEG is bonded to the surface thereof and rHSA is bonded to PEG terminus

### Method 1: Production wherein PEG-modified liposome is produced and then rHSA is bonded to PEG

### (1) Production of maleimide-PEG-modified liposome

About 8 mL of chloroform solution in which lipid was dissolved (total lipid: 100 µmol; dissolved in a molar ratio of egg yolk lecithin : cholesterol : maleimide-PEG-bonded DSPE (manufactured by Shearwater Co.) = 63 : 32 : 5) was added to an eggplant type flask. [³H (tritium)] cholesteryl hexadecyl ether (manufactured by Japan Radioisotope Association) (2,000,000 dpm) was added thereto. Chloroform was added so as to make a total volume of 10 mL and, after that, the solvent was evaporated *in vacuo* using a rotary evaporator under a nitrogen atmosphere followed by drying overnight. To the resulting thin film of lipid was added about 2 mL of PBS and the mixture was mixed with stirring for 15 minutes or longer by heating at 55°C so that the thin film of lipid was suspended. Particle size of the suspension was made uniform using an extruder equipped with a polycarbonate membrane of 200 nm.

### (2) Production of thiolated rHSA using SATA

8 µmol of SATA dissolved in dimethylformamide was added to an aqueous solution of rHSA (1 µmol) in such a manner that the concentration of dimethylformamide did not reach 1% or higher, and the mixture was shaken at room temperature for 30 minutes so that acetyl thioacetate was bonded to an amino group of rHSA. In order to remove the unreacted SATA, a gel filtration was conducted to collect an acetyl thioacetate-bonded rHSA fraction. Hydroxylamine (50 µmol) dissolved in 0.5 M HEPES and 25 mM EDTA was added to remove the acetyl group, thereby to produce a thiolated rHSA.

### (3) Bonding of modified PEG and rHSA to liposome

A maleimide-PEG-modified liposome solution and a solution of the thiolated rHSA were mixed and reacted at 4°C or lowers for 18 hours. Finally, gel filtration was conducted to remove the unreacted fraction of thiolated rHSA, thereby to recover a PEG-modified liposome to which rHSA was bonded.

### Method 2: Production method by bonding rHSA to PEG-DSPE followed by insertion into liposome

### (1) Production of liposome

About 8 mL of chloroform solution in which lipid was dissolved (total lipid: 95 µmol; dissolved in a molar ratio of egg yolk lecithin : cholesterol = 63 : 32) was added to an eggplant type flask. [³H (tritium)] cholesteryl hexadecyl ether (manufactured by Japan Radioisotope Association) (2,000,000 dpm) was added. Chloroform was added thereto so as to make a total volume of 10 mL and, after that, the solvent was evaporated *in vacuo* using a rotary evaporator under a nitrogen atmosphere, and dried overnight. To the resulting thin film of lipid was added about 2 mL of PBS, and the mixture was mixed with stirring for 15 minutes or longer by heating at 55°C so that the thin film of lipid was suspended. Particle size of the suspension was made uniform using an extruder equipped with a polycarbonate membrane of 200 nm.

### (2) Production of thiolated rHSA using SATA

8 µmol of SATA dissolved in dimethylformamide was added to an aqueous solution of rHSA (1 µmol) in such a manner that concentration of dimethylformamide did not reach 1% or higher, and the mixture was shaken at room temperature for 30 minutes so that acetyl thioacetate was bonded to an amino group of rHSA. In order to remove the unreacted SATA, a gel filtration was conducted to collect an acetyl thioacetate-bonded rHSA fraction. Hydroxylamine (50 µmol) dissolved in 0.5 M HEPES and 25 mM EDTA was added to eliminate the acetyl group, thereby to produce a thiolated rHSA.

### (3) Bonding of rHSA to maleimide-PEG

A maleimide-PEG-bonded DSPE (manufactured by Shearwater Co.) (5 µmol) and a solution of the thiolated rHSA were mixed at 4°C or lower and allowed to react for 18 hours. Gel filtration was conducted to collect a fraction of rHSA-PEG-bonded DSPE.

### (4) Insertion of rHSA-PEG-bonded DSPE into liposome

A solution of r-HAS-PEG-bonded DSPE was added to the liposome prepared in the above (1), and the mixture was shaken to be inserted into liposome, thereby to modify the surface of the liposome with rHSA-PEG.

### Example 3

### Production method for liposome where rHSA is bonded on the surface and, further, PEG is bonded to rHSA

### Method 1: Production using SATA and WSC

### (1) Production of NGPE-containing liposome

About 8 mL of chloroform solution in which lipid was dissolved (total lipid: 100 µmol; dissolved in a molar ratio of egg yolk lecithin : cholesterol : NGPE = 60 : 30 : 10) was added to an eggplant type flask. [³H (tritium)] cholesteryl hexadecyl ether (manufactured by Nippon Isotope Association) (2,000,000 dpm) was added. Chloroform was added thereto so as to make a total volume of 10 mL and, after that, the solvent was evaporated *in vacuo* using a rotary evaporator under a nitrogen atmosphere, and dried overnight. To the resulting thin film of lipid was added about 2 mL of PBS and the mixture was mixed with stirring for 15 minutes or longer by heating at 55°C so that the thin film of lipid was suspended. Particle size of the suspension was made uniform using an extruder equipped with a polycarbonate membrane of 200 nm.

### (2) Production of thiolated rHSA using SATA

8 µmol of SATA dissolved in dimethylformamide was added to an aqueous solution of rHSA (1 µmol) in such a manner that the concentration of dimethylformamide did not reach 1% or higher, and the mixture was shaken at room temperature for 30 minutes so that acetyl thioacetate was bonded to an amino group of rHSA. In order to remove the unreacted SATA, gel filtration was conducted to collect an acetyl thioacetate-bonded rHSA fraction. Hydroxylamine (50 µmol) dissolved in 0.5 M HEPES and 25 mM EDTA was added to eliminate the acetyl group, thereby to produce a thiolated rHSA.

### (3) Modification of rHSA to maleimide-PEG

A maleimide-PEG (manufactured by Shearwater Co.) (5 µmol) and a solution of the thiolated rHSA were mixed at 4°C or lower and allowed to react for 18 hours. Gel filtration was conducted to collect a rHSA-PEG fraction.

### (4) Bonding of rHSA-PEG to NGPE-containing liposome

100 µmol of NHS and 10 µmol of WSC were added to the above NGPE-containing liposome, and the mixture was shaken for 15 minutes so that NGPE and WSC were bonded thereto. To the solution was added 500 µmol of 2-mercaptoethanol. In order to remove NHS and WSC which were not bonded to the liposome, separation was conducted by gel filtration and a liposome fraction was recovered. rHSA-PEG (corresponding to 1 µmol of rHSA) was added immediately followed by shaking overnight so that rHSA-PEG was bonded to the liposome. Gel filtration was conducted for removal of the unreacted rHSA-PEG to recover an rHSA-PEG-bonded liposome fraction.

### Method 2: Production using SATA and SPDP

### (1) Production of DTP-DOPE

SPDP (12 µmol) was added to a solution of DOPE in 10 µmol of chloroform, and the mixture was stirred. To the reaction was added about 2 mL of PBS, and the mixture was vigorously stirred for 5 minutes and centrifuged at 3,000 rpm for 10 minutes. After removal of the PBS layer, pure water was added and the mixture was vigorously shaken for 3 minutes and centrifuged to remove the aqueous layer again. Washing with pure water as such was conducted once again. A white semi-solid remaining in the lower layer was subjected to solvent evaporation using an evaporator and then dried. 1.0 mL of chloroform was added to the residue to be redissolved, producing DTP-DOPE.

### (2) Production of DTP-DOPE-containing liposome

About 8 mL of chloroform solution in which lipid was dissolved (total lipid: 90 µmol; dissolved in a molar ratio of egg yolk lecithin : cholesterol = 60 : 30) was added to an eggplant type flask. After that, 10 µmol of DTP-DOPE was added thereto and 2,000,000 dpm of [³H (tritium)] cholesteryl hexadecyl ether (manufactured by Japan Radioisotope Association) was added to the mixture. Chloroform was added so as to make a total volume of 10 mL and then the solvent was evaporated *in vacuo* using a rotary evaporator under a nitrogen atmosphere, and dried overnight. To the resulting thin film of lipid was added about 2 mL of PBS and the mixture was mixed with stirring for 15 minutes or longer by heating at 55°C so that the thin film of lipid was suspended. Particle size of the suspension was made uniform using an extruder equipped with a polycarbonate membrane of 200 nm.

### (3) Production of thiolated rHSA using SATA

To an aqueous solution of rHSA (1 µmol) was added 8 µmol of SATA dissolved in dimethylformamide within such an extent that concentration of dimethylformamide did not reach 1% or higher, and the mixture was shaken at room temperature for 30 minutes so that acetyl thioacetate was bonded to an amino group of rHSA. In order to remove the unreacted SATA, gel filtration was conducted to collect an acetyl thioacetate-bonded rHSA fraction. Hydroxylamine (50 µmol) dissolved in 0.5 M HEPES and 25 mM EDTA was added to remove the acetyl group, thereby to produce a thiolated rHSA.

### (4) Modification of rHSA to maleimide-PEG

A maleimide-PEG (5 µmol) and a solution of the thiolated rHSA were mixed at 4°C or lower and allowed to react for 18 hours. Gel filtration was conducted to collect a rHSA-PEG fraction.

### (5) Production of thiolated rHSA-PEG

SPDP (200 µmol) was added to an aqueous solution of rHSA-PEG (corresponding to 1 µmol of rHSA), and the mixture was stirred to produce DTP-rHSA-PEG. Gel filtration was conducted to remove the unreacted SPDP to collect a DTP-rHSA-PEG fraction. DTT was added to the DTP-rHSA-PEG fraction so as to make a final concentration of 50 mM, and the mixture was stirred for 20 minutes so that a part of rHSA was thiolated. In order to remove the unreacted DTT, gel filtration was conducted to collect a fraction of the thiolated rHSA-PEG.

### (6) Reaction of DTP-DOPE-containing liposome with thiolated rHSA-PEG

To a DTP-DOPE-containing liposome was added a solution of the thiolated rHSA-PEG, and the mixture was stirred at room temperature for 24 hours or longer so that the liposome was modified with rHSA-PEG. After that, the reaction solution was subjected to gel filtration to separate liposome and the unreacted rHSA-PEG whereby a liposome fraction was collected and rHSA-PEG-modified liposome was prepared.

### Test Example. Experiment of administration of a liposome sample to rats

### (1) Investigation of blood level

A liposome sample (20 µmol/kg) prepared in Example 1 was administered to each of three rats. From immediately after the administration to 24 hours thereafter, about 300 µL of blood was collected every 4 hours from the carotid artery and immediately subjected to a centrifugal separation (at 1500 x g at 4°C for 3 minutes). A supernatant liquid (100 µL) was recovered, 10 mL of Clearsol (liquid scintillation cocktail) was added thereto, and the mixture was mixed well. The liquid was quantified using a liquid scintillation counter. Measurement was conducted for 5 minutes for each sample.

As a comparison, the same tests were conducted using liposome where no modification was done on its surface and using liposome of which surface was modified with PEG.

The results are shown in Fig. 1. The data shown in Fig. 1 are mean values of blood level in three rats.

### Industrial Applicability

When polyethylene glycol chain and albumin molecule are bonded to the surface of liposome, it is now possible that retentivity of liposome in blood upon administration to humans and animals is improved and that therapeutic effect and diagnostic effect of a drug which is included in or bonded to liposome are enhanced. By using genetically recombinant human serum albumin as albumin, it is now possible to prepare a liposome having no risk of infection and having an improved biocompatibility in terms of metabolism as compared with a case of single PEG.

## Claims

1. A liposome to which a polyalkylene glycol and albumin are bonded.

2. The liposome according to claim 1, wherein a physiologically active ingredient is further contained.

3. The liposome according to claim 2, wherein the physiologically active ingredient is a pharmaceutically active ingredient.

4. The liposome according to claim 3, wherein the pharmaceutically active ingredient is an antitumor agent.

5. A pharmaceutical composition containing the liposome mentioned in any one of claims 2 to 4.

6. The pharmaceutical composition according to claim 5, which is an injection.

7. A method for treatment of cancer, which comprises administering a pharmaceutical composition comprising a liposome to which a polyalkylene glycol and albumin are bonded and in which an antitumor agent is contained.

8. Use of a liposome to which a polyalkylene glycol and albumin are bonded and in which a physiologically active ingredient is contained, for the extension of the in vivo retention time of the physiologically active ingredient.

9. A process for the production of the liposome mentioned in claim 1, **characterized in that**,
a liposome having a compound represented by the following formula (1): 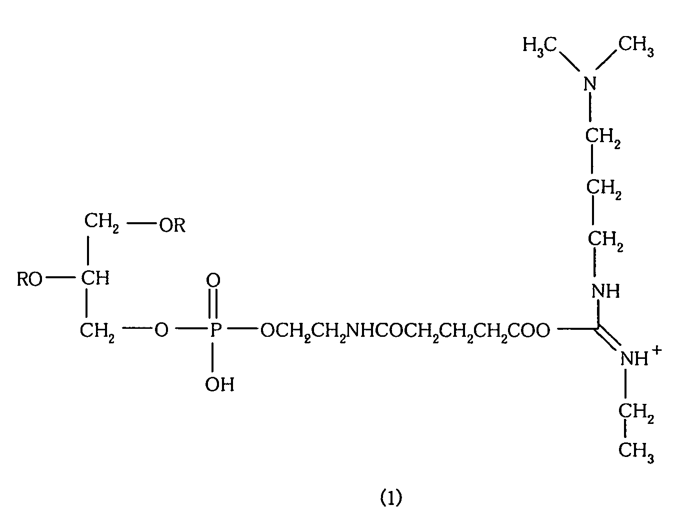 (wherein R is an acyl group derived from a fatty acid having 2 to 35 carbon atoms) as a constituent lipid is bonded to albumin;
a liposome having a compound represented by the following formula (2): 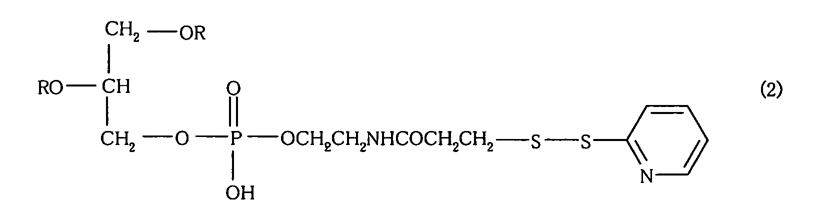 (wherein R has the same meaning as defined above) as a constituent lipid is bonded to a compound represented by the formula (3):
(Alb-NH)-CO-CH₂-CH₂-SH (3)
(wherein Alb-NH is a group formed by removing one hydrogen atom of the amino group from an albumin molecule represented by Alb-NH₂);
a liposome having a compound represented by the following formula (4): 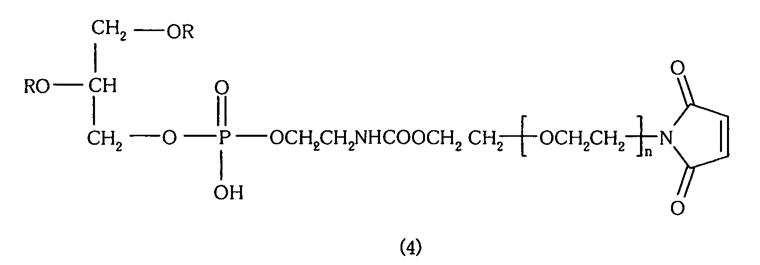 (wherein n is an integer of 5 to 100,000 and R has the same meaning as defined above) as a constituent lipid is bonded to a compound represented by the formula (5):
(Alb-NH)-CO-CH₂-SH (5)
(wherein Alb-NH has the same meaning as defined above);
a compound represented by the following formula (6): 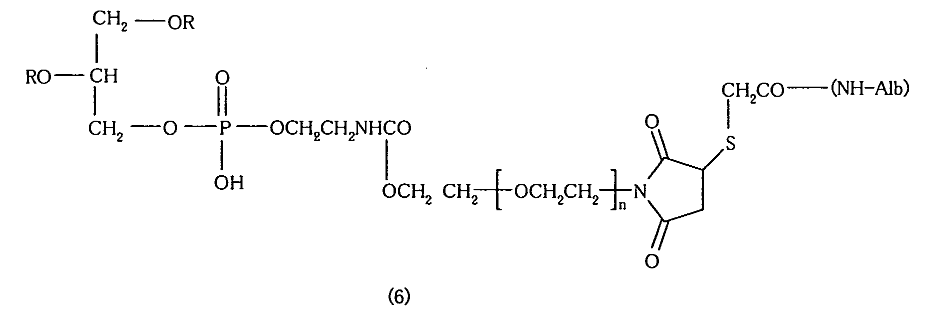 (wherein n, R and Alb-NH have each the same meaning as defined above) is inserted into a liposome;
a liposome having the compound represented by the above formula (1) as a constituent lipid is bonded to a compound represented by the following formula (7): 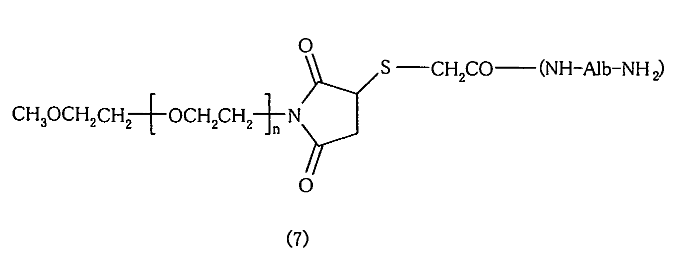 (wherein -NH-Alb-NH₂ is a group formed by removing one hydrogen atom from the one amino group of an albumin molecule represented by H₂N-Alb-NH₂, and n has the same meaning as defined above); or
a liposome having the compound represented by the above formula (2) as a constituent lipid is bonded to a compound represented by the following formula (8): 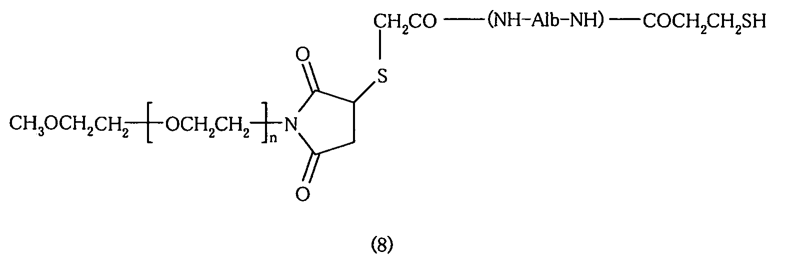 (wherein -NH-Alb-NH- is a group formed by removing each one hydrogen atom from the two amino groups of an albumin molecule represented by the formula H₂N-Alb-NH₂, and n has the same meaning as defined above).
